# EUROPEAN PATENT APPLICATION

(11) **EP 3 811 923 A1**
(43) Date of publication of application: **28.04.2021**
(21) Application number: 19205501.0
(22) Date of filing: 25.10.2019
(51) Int. Cl.: A61K 8/362, A61K 8/46, A61Q 5/00, A61Q 5/02, A61K 8/365

(54) **CLEANSING COMPOSITIONS**

(71) Applicant: Unilever PLC, London Greater London EC4Y 0DY (GB)
(72) Inventor: The designation of the inventor has not yet been filed
(74) Representative: Chisem, Janet

(57) **Abstract**

A shampoo composition comprising
(i) an organic acid selected from butane tetra carboxylic acid, fumaric acid, tartaric acid and combinations thereof; and
(ii) a cleansing surfactant;
wherein the composition has a pH of from 3 to 6, provides repair to damaged internal protein of hair.

## Description

### FIELD OF THE INVENTION

The invention relates to the use of hair treatment compositions in the repair of hair damage.

### BACKGROUND AND PRIOR ART

Consumers regularly subject their hair to intensive treatment, and care and styling routines to help them achieve their desired look. The actions performed by consumers introduce modifications to the chemistry of hair keratin protein which results in micro- and macro-structural changes and, in turn, changes fibre physical properties: the consequences of these are generally perceived by the consumer as damage.

Combing and brushing of hair mechanically abrades the fibre cuticle making this rougher and increasing the frictional characteristics. Hair lightening, such as bleaching, or colouring treatments generally involve an oxidative step to break down melanin and develop the new hair colour, but these processes also oxidise the hair fibre protein and the endogenous lipids. These reactions alter the number and types of covalent and noncovalent bonds within the fibre and impact the thermal stability and mechanical properties of the hair. The internal protein of damaged hair typically has a reduced denaturation temperature compared to that of virgin hair.

Various organic acids, organic molecules and combinations thereof have been suggested for use in the treatment of damaged hair.

WO 2004054526 describes hair treatment compositions for the care and repair of damaged hair, and for improving hair manageability, comprising a disaccharide and a di-acid, especially succinic acid and adipic acid.

WO2005/094761 discloses a combination of a disaccharide, a di-acid, particularly succinic acid and adipic acid and a source of ammonium ions, preferably ammonium carbonate to deliver styling, smoothing, alignment and damage prevention benefits to hair.

WO2005/097048 discloses a hair treatment composition such as a shampoo or conditioner comprising a disaccharide, alkali metal salt and a di-acid or salt thereof. Use of said composition for smoothing and aligning hair, for preventing damage to the hair is disclosed.

WO2016/188691, WO2017/157993 and WO2019/030034 disclose the use of a hair treatment composition comprising a lactone, a disaccharide, an inorganic salt and an organic acid or salt thereof, having a pH in the range of from 3 to 6.5, in the treatment of hair, to repair damage to hair protein. Suitable acids include glycolic acid, lactic acid, citric acid, mandelic acid, propanoic acid, betahydroxypropionic acid, betahydroxybutyric acid, salicylic acid, carnitine and mixtures thereof.

### DEFINITION OF THE INVENTION

In a first aspect of the invention, there is provided a shampoo composition comprising
(i) an organic acid selected from butane tetra carboxylic acid, fumaric acid, tartaric acid and combinations thereof; and
(ii) a cleansing surfactant;
wherein the composition has a pH of from 3 to 6.

The present invention provides damage repair to hair, preferably an increase in the denaturation temperature of the internal protein of hair.

A second aspect of the invention provides a method of treating hair, comprising the step of applying a shampoo composition of the first aspect.

Preferably, the method provides a step of rinsing the composition from the hair.

A use is also provided, of a composition comprising an organic acid selected from butane tetra carboxylic acid, fumaric acid, tartaric acid and combinations thereof and a cleansing surfactant to repair damage to hair protein.

### The Hair

The hair is preferably damaged hair, where the denaturation temperature of the internal protein of the hair is reduced.

The damage may be caused by mechanical means, for example combing and brushing, chemical means, exposure to heat, environmental means such as sunlight and exposure to damaging energy sources, for example light such as UV light. Chemical means includes treatments that involve an oxidative step, for example, hair lightening, such as bleaching, and colouring treatments. Preferably the hair is bleached, more preferably bleached multiple times.

### GENERAL DESCRIPTION OF THE INVENTION

### Shampoos

The shampoo compositions of the invention have a pH of from 3 to 6, preferably 3 to 5.

Shampoo compositions of the invention are generally aqueous, i.e. they have water or an aqueous solution or a lyotropic liquid crystalline phase as their major component.

Suitably, the shampoo composition will comprise from 50 to 98%, preferably from 60 to 90% water by weight based on the total weight of the composition.

Shampoo compositions according to the invention comprise one or more cleansing surfactants which are cosmetically acceptable and suitable for topical application to the hair. Preferably, the cleansing surfactant is an anionic surfactant.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic cleansing surfactants are sodium lauryl sulphate, sodium lauryl ether sulphate (n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5 to 45%, preferably from 1.5 to 35%, more preferably from 5 to 20% by total weight anionic cleansing surfactant based on the total weight of the composition.

Optionally, a shampoo composition of the invention may contain further ingredients as described below to enhance performance and/or consumer acceptability.

The composition can include co-surfactants, to help impart aesthetic, physical or cleansing properties to the composition.

An example of a co-surfactant is a nonionic surfactant, which can be included in an amount ranging from 0.5 to 8%, preferably from 2 to 5% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco mono-isopropanolamide. Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C_{I}-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 8%, preferably from 1 to 4% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1 to 50%, preferably from 2 to 40%, more preferably from 10 to 25% by total weight surfactant based on the total weight of the composition.

Cationic polymers are preferred ingredients in a shampoo composition of the invention for enhancing conditioning performance.

Suitable cationic polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15 and JAGUAR C17.

Mixtures of any of the above cationic polymers may be used.

Cationic polymer will generally be present in a shampoo composition of the invention at levels of from 0.01 to 5%, preferably from 0.05 to 1%, more preferably from 0.08 to 0.5% by total weight of cationic polymer based on the total weight of the composition.

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

### Acids:

The compositions of the present invention comprise an organic acid selected from butane tetra carboxylic acid, fumaric acid, tartaric acid and combinations thereof. Preferably the organic acid is tartaric acid. Preferably the amount of organic acid is in the range of from 0.01 to 5 wt. %, by weight of the total composition.

### Form of Composition

Hair treatment compositions according to the invention, particularly water-based shampoos, will preferably also contain one or more silicone conditioning agents.

Particularly preferred silicone conditioning agents are silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC2-8177 and DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula:

HO(CH₂CH₂O)ₓ(CH(CH₃)CH₂O)_{y}(CH₂CH₂O)ₓ H

wherein the mean value of x is 4 or more and the mean value of y is 25 or more.

Another preferred form of the surface active block copolymer is according to the following formula:

(HO(CH₂CH₂O)ₐ(CH(CH₃)CH₂O)_{b})₂-N-CH₂-CH₂-N((OCH₂CH(CH₃))_{b}(OCH₂CH₂)ₐOH)₂

wherein the mean value of a is 2 or more and the mean value of b is 6 or more.

Mixtures of any of the above described silicone emulsions may also be used.

The above described silicone emulsions will generally be present in a composition of the invention at levels of from 0.05 to 10%, preferably 0.05 to 5%, more preferably from 0.5 to 2% by total weight of silicone based on the total weight of the composition.

### Other Ingredients

A composition of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally, these optional ingredients are included individually at a level of up to 5% by weight of the total composition.

Hair treatment compositions of the invention are primarily intended for topical application to the hair and/or scalp of a human subject, either in rinse-off or leave-on compositions, for the treatment of dry, damaged and/or unmanageable hair.

The invention will be further illustrated by the following, non-limiting Example, in which all percentages quoted are by weight based on total weight unless otherwise stated.

### EXAMPLES

### The Hair

Virgin: The hair used in the following examples was dark brown European hair tresses 2.5 grams and 6 inches long.

Bleached: Virgin hair tresses were bleached according to the following protocol. Hair was bleached for 30 minutes with commercial bleaching powder mixed with 9% cream peroxide, 30 'vol'. Hair was then washed with 14% aqueous sodium laureth ether sulphate (SLES) solution before drying.

The bleached hair was dialysed prior to the experiments in 5L of distilled water over a period of 72 hours, and the water was changed 3 times over this period. After dialysing, the hair tresses were left to dry overnight in a controlled environment (20°C and 50% relative humidity).

### Examples:

Hair damage repair can be measured by the denaturation temperature of the hair fibre. Without wishing to be bound by theory, it is believed that some acids can penetrate the hair fibre and help to re-build damaged protein structures. This in turn leads to the increased denaturation temperature of the fibre. Therefore, higher denaturation temperature indicates damage repair in a fibre.

### Denaturation temperature (Td) Model:

A reliable denaturation temperature (Td) model has been used to investigate which acids provide damage repair to hair fibres via one shampoo wash at pH 4.5 (i.e. which acids increase the denaturation temperature of the hair fibre). The model calculates Td values for acids based on: % level of acid, pKa of the acid, molecular weight, number of rotatable bonds, number of hydrogen bond acceptors, number of hydrogen bond donners, partition coefficient Alog P, solubility measured as log S and Apol.

The model has been validated against measured Td values, see Table 1.

### Physical measurement of Td:

Twice bleached hair was washed once with a shampoo containing acid at 1% level at pH 4.5 (1 wash = 2 shampoos applications)

Measurements were performed using a Mettler-Toledo DSC (with auto-sampler). 7-10 mg samples of dry, finely chopped hair was placed in the 'Medium Pressure Stainless Steel DSC Pans' and accurately weighed. 50 microlitres of deionised water was then added to each sample after which the pan lid was put on and the pans crimped shut to provide a hermetic seal. Pans were equilibrated for a minimum of 24 h ahead of any measurement to allow the hair to fully hydrate. The DSC was programmed to first heat each sample to 100 °C for 3 min and then to warm them further from 100 to 180 °C at a constant rate of 5 °C min'.

**Table 1 - Comparison of Measured Td and Predicted Td at 1 wt.%:**

| | Measured Td | Predicted Td |
|---|---|---|
| Tartronic acid | 144.9 | 144.8 |
| Gluconic acid | 146.75 | 146.8 |
| Itaconic acid | 148.0 | 148.0 |
| Untreated hair | 148.0 | - |
| Acetic acid | 148.15 | 148.2 |
| Malonic acid | 148.8 | 148.25 |
| Malic acid | 148.9 | 148.25 |
| Butane Tetra Carboxylic acid | - | 148.8 |
| Fumaric acid | - | 149.2 |
| Tartaric acid | - | 149.6 |

Using the denaturation temperature model, treatment with butane tetra carboxylic acid, fumaric acid and tartaric acid are identified as providing the highest denaturation temperature, i.e. the best damage repair.

**Example formulation:**

| **Ingredient** | **Amount (wt. %)** |
|---|---|
| Acid¹ | 1 |
| Suspending agent | 0.4 |
| Sodium Laureth Ether Sulfate | 12.0 |
| Co-surfactant | 1.6 |
| Deposition aid | 0.2 |
| Silicone | 0.75 |
| Sodium Chloride | 1.0 |
| Perfume | 0.70 |
| Water & minors | to 100 |
| pH | 4.5 |

| | |
|---|---|
| Acid¹ - in accordance with the present invention | |

## Claims

1. A shampoo composition comprising
(i) an organic acid selected from butane tetra carboxylic acid, fumaric acid, tartaric acid and combinations thereof; and
(ii) a cleansing surfactant;
wherein the composition has a pH of from 3 to 6.

2. The composition as claimed in claim 1, wherein the organic acid is tartaric acid.

3. The composition as claimed in any preceding claim, wherein the amount of organic acid is in the range of from 0.01 to 5 wt. %, by weight of the total composition.

4. The composition as claimed in any preceding claim, wherein the cleansing surfactant is an anionic surfactant.

5. The composition as claimed in any preceding claim, wherein the pH is from 3 to 5.

6. A method of treating hair, comprising the step of applying a composition as claimed in any preceding claim, to hair.

7. A method as claimed in claim 6, wherein the hair is bleached.

8. Use of a composition comprising an organic acid selected from butane tetra carboxylic acid, fumaric acid, tartaric acid and combinations thereof, to repair damage to hair protein.

9. Use as claimed in claim 8, wherein the composition is a shampoo composition, further comprising a cleansing surfactant.

## Amended claims

### Amended claims in accordance with Rule 137(2) EPC.

1. A shampoo composition comprising
(i) 0.01 to 5 wt % of an organic acid selected from butane tetra carboxylic acid a combination of butane tetra carboxylic acid with fumaric acid and a combination of butane tetra carboxylic acid with tartaric acid;
(ii) 0.5 to 45 wt % of a cleansing surfactant; and
(iii) a silicone emulsion;
wherein the composition has a pH of from 3 to 6.

2. The composition as claimed in claim 1, wherein the organic acid is butane tetra carboxylic acid.

3. The composition as claimed in any preceding claim, wherein the amount of cleansing surfactant is in the range of from 5to 20 wt. %, by weight of the total composition.

4. The composition as claimed in any preceding claim, wherein the cleansing surfactant is an anionic surfactant.

5. The composition as claimed in any preceding claim, wherein the pH is from 3 to 5.

6. The composition as claimed in any preceding claim, wherein the silicone emulsion is present in an amount of from 0.5 to 2 wt %.

7. A method of treating hair, comprising the step of applying a composition as claimed in any preceding claim, to bleached hair.

8. Use of a composition comprising as defined in any of claims 1 - 6 to repair damage to hair protein, wherein the hair is bleached hair.
